## Europäisches Patentamt

## European Patent Office

## Office européen des brevets

(19)

(11) Veröffentlichungsnummer : **0 417 501 B1**

(12)

# EUROPÄISCHE PATENTSCHRIFT

(45) Veröffentlichungstag der Patentschrift :
**15.12.93 Patentblatt 93/50**

(51) Int. Cl.⁵ : **A61K 7/50, A61K 7/075**

(21) Anmeldenummer : **90115816.2**

(22) Anmeldetag : **17.08.90**

(54) **Extra-milde Duschgel- und Haarschampoo-Formulierung mit niedriger Tensidkonzentration.**

(30) Priorität : **14.09.89 DE 3930725**

(43) Veröffentlichungstag der Anmeldung :
**20.03.91 Patentblatt 91/12**

(45) Bekanntmachung des Hinweises auf die
Patenterteilung :
**15.12.93 Patentblatt 93/50**

(84) Benannte Vertragsstaaten :
**AT BE CH DE DK ES FR GB GR IT LI LU NL SE**

(56) Entgegenhaltungen :
**EP-A- 0 099 987**
**EP-A- 0 384 982**
**DE-A- 2 847 439**
**DE-A- 3 414 090**
**US-A- 3 990 991**

(73) Patentinhaber : **Joh. A. Benckiser GmbH**
**Postfach 21 10 67, Ludwig-Bertram-Strasse 8**
**+ 10**
**D-67059 Ludwigshafen (DE)**

(72) Erfinder : **Baust, Heinrich**
**Westende 29**
**D-6831 Plankstadt (DE)**

(74) Vertreter : **Grussdorf, Jürgen, Dr. et al**
**Patentanwälte Zellentin & Partner**
**Rubensstrasse 30**
**D-67061 Ludwigshafen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 417 501 B1

## Beschreibung

Gegenstand der vorliegenden Erfindung ist eine neue extramilde Duschgel- und Haarshampoo-Formulierung mit niedriger Tensidkonzentration.

Die Produktionsmenge für Duschbäder und Haarshampoos der Kosmetikindustrie in Deutschland liegt derzeit bei ca. 110.000 jato. Der Tensidanteil in den handelsüblichen Produkten bewegt sich dabei zwischen 15 und 25 %. So gelangen ca. 20.000 jato Tenside in die Abwässer, wo sie mehr oder weniger schnell und vollständig abgebaut werden. Eine Verringerung der waschaktiven Substanzen ist sowohl aus ökonomischer als auch ökologischer Sicht ein erstrebenswertes Ziel.

Es ist bekannt, daß in ethersulfathaltigen Formulierungen durch deren starke Schaumbildung eine drastische Senkung der Waschaktiven-Substanzen von z. B. 20 % auf 10 % und darunter möglich ist und der eintretende Viskositätsabfall durch die Abwässer belastende-Elektrolytzugaben(NaCl, $Na_2SO_4$) kompensiert werden kann. Die Salzbelastung übersteigt dabei leicht die Tensid-Konzentration, was einerseits das Risiko des Augenbrennens, andererseits das der Instabilität (Aussalzungseffekt) deutlich erhöht. Unabhängig davon enthalten ethersulfathaltige Tenside in Spuren das pharmakologisch bedenkliche Dioxan.

Bekannte ethersulfatfreie Tensidsysteme können bei niedrigen Tensidkonzentrationen (10 % und darunter) durch Elektrolytenzugaben nicht ausreichend verdickt werden. Diese Systeme benötigen daher zusätzliche Verdicker, die die Produkteigenschaften (Schaumstruktur, Konditionierung etc,) und Kosten negativ beeinflussen. Ganz allgemein kann man davon ausgehen, daß ethersulfatfreie Tensidkombinationen kein ausreichendes Verdickungsvermögen trotz Salzbelastungen aufweisen und zu den schwachen Schäumern zählen.

In der nicht vorpublizierten älteren EP-A 0 384 982 ist eine Detergentienzusammensetzung mit erhöhter Viskosität bekannt, die eine Mischung von Alkyl- und Alkylenpolyglykolethermethylencarboxylaten mit Elektrolyten und ggf. Hilfstensiden darstellt, welche weitgehend frei von Alkylbzw. Alkylethersulfaten, sowie N-haltigen Tensiden ist.

Aus der DE-A 34 14 090 ist ein waschaktives Mittel bekannt, welches u.a. eine Kombination aus Polyglykolethercarbonsäuren sowie ggf. Alkylsulfaten mit anionischen Polymeren als Verdickern beschreibt. Höhere Ethoxylierungsgrade von 5-25 werden wegen der geringeren Irritation für notwendig gehalten.

Aus der EP-A 0 099 987 sind Haarschampoos bekannt, bei denen beliebige, mit Ausnahme von kationischen Tenside auf Schichtsilikate adsorbiert sind, welche als Verdicker wirken und die Splißrate der Haare vermindern. Eine verdickende Wirkung spezieller Tensidkombinationen ist dabei nicht angesprochen.

Es stellte sich daher die Aufgabe, eine ethersulfatfreie, milde und gut schäumende Tensidkombination für die Hautund Haarpflege zu finden, mit der bei Tensidkonzentrationen von unter 10 % hochviskose Zubereitungen mit einem Minimum an Elektrolytsalzen und ohne zusätzliche Verdicker hergestellt werden können.

Überraschenderweise wird diese Aufgabe durch die in den Ansprüchen näher gekennzeichneten Merkmale gelöst, beziehungsweise gefördert.

Aus der älteren, nicht vorveröffentlichten Anmeldung, P 39 10 652.7 ist bekannt, daß eine Tensidkombination aus Laurylpolyglykolethercarboxylat, Laurylsulfat und Laurinsäureamidopropylbetain mit 2 - 10 % Natriumchlorid zu einem gut verträglichen Duschgel verarbeitet werden kann. Das dabei verwendete Laurylglykolethercarboxylat hatte einen Ethoxylierungsgrad von 8 - 10 Ethoxygruppen. Um ein ausreichendes Schaumverhalten zu erzeugen, war eine Gesamttensid-Konzentration von 15 - 20 % erforderlich. Zur Viskositätseinstellung wurden 4,5 % NaCl zugesetzt.

Überraschenderweise zeigen die bei alleiniger Verwendung agressiveren Alkyl-ethercarbonsäuren mit niederem Ethoxylierungsgrad (2 - 5) in der erfindungsgemäßen Kombination keine Hautirritationen mehr und weisen ein so starkes Schaumbildungsvermögen auf, daß Gesamttensid-Konzentrationen von unter 10 % möglich sind.

Alkylpolyglykolethercarboxylate im Sinne der Erfindung sind Verbindungen der Formel I

$$R-O-(CH_2 - CH_2 - O)_n - CH_2 - CO_2 (-) \qquad (I)$$

in der R eine Alkylgruppe mit bis zu 20 C-Atomen, vorzugsweise 10 - 16, insbesondere 12 - 14 C-Atomen, und n eine natürliche Zahl von 2 - 5 bedeutet. Die Carboxylate sind vorzugsweise mit Natrium, Kalium oder Ammoniumionen, insbesondere Tris( hydroxymethyl)-aminomethan neutralisiert.

Bei der Poly-Kondensation der Glykoleinheiten entstehen Gemische, die für den Einsatz als Tensid nicht oder nur grob getrennt werden. Die Zahl n ist daher als Mittelwert der enthaltenen Verbindungen zu verstehen.

Alkylsulfate im Sinne der Erfindung sind als Tenside gut bekannte von Fettalkoholen abgeleitete Monoalkylschwefelsäurester, wobei wegen des besonders guten Schaumverhaltens das Laurylsulfat bevorzugt wird.

Fettsäureamidopropylbetaine sind ebenfalls als Tenside gut bekannt. Als Fettsäuren sind solche mit 8 - 20 C-Atomen üblich. Wegen des optimalen Schaumverhaltens und der Zusammenwirkung mit den beiden anderen Komponenten im Sinne einer guten Verdickung, ohne daß ein schwerlöslicher Niederschlag entsteht, wird Laurylamidopropylbetain bevorzugt.

Ein weiterer Vorteil der erfindungsgemäßen Mischungen liegt darin, daß sie eiweißfrei formuliert werden können, da auch die hierbei bekannten Aminosäurengemische allergen sein können. Als Rückfettungskomponente wird die Verwendung von Laurinsäuremonoglycerid zusammen mit der erfindungsgemäßen Tensidmischung vorgeschlagen.

Zur Solubilisierung von üblicherweise in derartigen Hautreinigungsmitteln eingesetzten Parfümölen hat sich der Einsatz von Laurylalkoholethoxylat besonders bewährt, da dieses Mittel Öl besonders gut löslich macht.

Ein Duschgel, wie es bevorzugt mit Hilfe der vorgenannten Substanzen herstellbar ist, enthält neben dem schon genannten Parfümöl Kochsalz als Verdickungsmittel und/oder den polyfunktionalen Wirkstoff Na-Hexametaphosphat. Des weiteren Substanzen zur Konservierung und Wasser.

Erfindungsgemäß zusammengesetzte Duschgele weisen vorzugsweise die nachstehenden Mengenverhältnisse (Gew. %) auf:

```
Alkylpolyglycolether-carboxylat- Tris-Salz   2    -   4,5
Natriumlaurylsulfat                        1,5    -   3,5
Fettsäure-amidopropyl-betain               1,5    -     3
Eiweißhydrolysat                           0,3    -   1,5
Laurylalkohol-ethoxylat                    0,1    -   0,5
Polyquaternium (Merquat 550)               0,2    -     2
Parfüm                                     0,5    -     2
Na-Hexametaphosphat                          0    -     5
NaCl                                         2    -    10
Konservierungsmittel                      0,05    -   0,5
Wasser                                          ad 100
```

Ausführungsbeispiele

Beispiel 1     10 % WAS-Duschgel

```
                                                            E %

C12/14 Ethercarbonsäure, 2,5 Mol EO, Na-Salz,    4,27
Natriumlaurylsulfat,                             3,25
Fettsäure-amidopropylbetain,                     2,53
Eiweißhydrolysat,                                0,91
Glycerinpartialester, ethoxyliert               0,32
Parfümöl                                         0,72
Farbstoffe                                       q.s.
Euxyl K 400                                      0,07
NaCl                                      ca.    2,00
Wasser, entkeimt                          ad      100
```

(R = Cocos- bzw. Lauryl-)

pH-Wert mit NaOH bzw. TRIS auf pH 6,5 - 6,8 eingestellt

Beispiel 2      8 % WAS-Duschgel

|  | | E % |
|---|---|---|
| C 12/14 Ethercarbonsäure, 2,5 Mol EO  Na-Salz, | | 3,39 |
| Natriumlaurylsulfat | | 2,58 |
| Fettsäure-amidopropylbetain | | 2,01 |
| Eiweißhydrolysat | | 0,72 |
| Glycerinpartialester, ethoxyliert | | 0,26 |
| Parfümöl | | 0,57 |
| Farbstoffe | | q.s. |
| Euxyl K 400 | | 0,07 |
| NaCl | ca. | 2,00 |
| Wasser, entkeimt | ad | 100 |

(R = Cocos-bzw. Lauryl-)
pH-Wert mit NaOH bzw. TRIS auf pH 6,5 - 6,8 eingestellt

Beispiel 3      10 % WAS-Haarshampoo

|  | | E % |
|---|---|---|
| C 12/14 Ethercarbonsäure, 2,5 Mol EO (Na-Salz) | | 4,27 |
| Natriumlaurylsulfat, | | 3,25 |
| Fettsäure-amidopropylbetain | | 2,53 |
| Eiweißhydrolysat | | 0,91 |
| Merquat 550 (Haarfestiger) | | 1,20 |
| Glycerinpartialester, ethoxyliert | | 0,32 |
| Parfümöl | | 0,50 |
| Farbstoffe | | q.s. |
| Euxyl K 400 | | 0.07 |
| NaCl | ca. | 2,00 |
| Wasser, entkeimt | ad | 100 |

(R = Cocos- bzw. Lauryl-)
pH-Wert mit NaOH bzw. TRIS auf pH 6,5 - 6,8 eingestellt

Beispiel 4     8% WAS-Haarshampoo

```
                                                    E %

C 12/14 Ethercarbonsäure, 2,5 Mol EO  Na-Salz    3,32
Natriumlaurylsulfat                              2,58
Fettsäure-amidopropylbetain                      2,01
Eiweißhydrolysat                                 0,72
Merquat 550 (Haarfestiger)                       1,00
Glycerinpartialester, ethoxyliert               0,26
Parfümöl                                         0,57
Farbstoffe                                       q.s.
Euxyl K 400                                      0,07
NaCl                                     ca.     2,00
Wasser, entkeimt                         ad      100
```

(R = Cocos-bzw. Lauryl-)
pH-Wert mit NaOH bzw. TRIS auf pH 6,5 - 6,8 eingestellt

## Patentansprüche

1.   Duschgel und Haarshampoo mit folgenden Merkmalen:
     a) Die Mischung ist eine wässrige Lösung;
     b) sie enthält eine neutralisierte Tensidkombination aus Alkylpolyglykolethercarboxylat, Alkylsulfat und Fettsäure-amidopropyl-betain, in einem Verhältnis von etwa 1,8 - 4, 3 : 1,5 - 3,2 :1;
     c) der mittlere Ethoxilierungsgrad der Alkylpolygly kolethercarboxylate beträgt 2 - 5;
     d) der Tensidgehalt ist kleiner gleich 10 Gew.-%;
     e) die Mischung enthält übliche Hilfs- und Trägerstoffe und Elektrolyte, mit Ausnahme von unlöslichen Feststoffen und Verdickungsmitteln.

2.   Duschgel und Haarshampoo nach Anspruch 1, in dem die ver wendete Alkylpolyglykolethercarbonsäure die allgemeine Formel I aufweist,

$$R\text{-}0\text{-}(CH_2CH_2O)_n \, CH_2COOH \qquad (I)$$

     in der R eine Alkylgruppe mit 10 - 16, insbesondere 12 oder 14 C-Atomen, und n im Mittel 2,5 bedeutet.

3.   Duschgel und Haarshampoo nach Anspruch 1 oder 2, in dem als Neutralisierungsmittel KOH, NaOH, vorzugsweise Tris-(hydroxymethyl)-aminomethan enthalten ist.

4.   Duschgel und Haarshampoo nach einem oder mehreren der Ansprüche 1 - 3, **gekennzeichnet** durch einen Gehalt an Laurylalkohol-ethoxylat.

5.   Duschgel und Haarshampoo nach einem oder mehreren der Ansprüche 1 - 4, **dadurch gekennzeichnet,** daß in ihm Conditionierer wie Eiweißhydrolysate und/oder Acrylamid-Polymere enthalten sind.

6.   Duschgel und Haarshampoo nach einem oder mehreren der Ansprüchen 1 - 5, **dadurch gekennzeichnet,** daß ein Elektrolyt, insbesondere Natriumchlorid, oder ein Polyphosphat, insbesondere Na-Hexametaphosphat als Verdicker enthalten ist.

7.   Duschgel und Haarshampoo nach einem oder mehreren der Ansprüche 1 - 6, **gekennzeichnet** durch fol-

5

gende Zusammensetzung in Gew. %

```
Alkylpolyglykolether-carboxylat Tris-Salz   2  -      4
Natriumlaurylsulfat                       1,5  -    3,5
Fettsäure-amidopropyl-betain              1,5  -      3
Eiweißhydrolysat                          0,3  -    1,5
Laurylalkohol-ethoxylat                   0,1  -    0,5
Polyquaternium (Merquat 550)              0,2  -      2
Parfüm                                    0,5  -      2
Na-Hexametaphosphat                         0  -      5
NaCl                                        2  -     10
Konservierungsmittel                     0,05  -    0,5
Wasser                                    ad 100
```

**Claims**

1. Shower gel and hair shampoo with the following characteristics:
   a) the mixture is an aqueous solution;
   b) it contains a neutralised tenside combination of alkyl polyglycol ether carboxylate, alkyl sulphate and fatty acid amidopropylbetaine in a ratio of about 1.8-4.3 : 1.5-3.2 : 1;
   c) the average degree of ethoxylation of the alkyl polyglycol ether carboxylate amounts to 2 - 5;
   d) the tenside content is smaller or equal than 10 wt.%;
   e) the mixture contains usual adjuvant and carrier materials and electrolytes with the exception of insoluble solid materials and thickening agents.

2. Shower gel and hair shampoo according to claim 1, in which the alkyl polyglycol ether carboxylic acid used has the general formula I
$$R\text{-}0\text{-}(CH_2\text{-}CH_20)_n\text{-}CH_2COOH \qquad (I)$$
in which R signifies an alkyl group with 10 - 16, especially 12 or 14 C-atoms and $\underline{n}$ signifies on average 2.5.

3. Shower gel and hair shampoo according to claim 1 or 2, in which KOH, NaOH, preferably tris-(hydroxymethyl)-aminomethane is contained as neutralising agent.

4. Shower gel and hair shampoo according to one or more of claims 1 - 3, characterised by a content of lauryl alcohol ethoxylate.

5. Shower gel and hair shampoo according to one or more of claims 1 - 4, characterised in that conditioners are contained in it, such as protein hydrolysates and/or acrylamide polymers.

6. Shower gel and hair shampoo according to one or more of claims 1 - 5, characterised in that an electrolyte, especially sodium chloride, or a polyphosphate, especially Na hexametaphosphate, is contained as thickener.

7. Shower gel and hair shampoo according to one or more of claims 1 - 6, characterised by the following composition in wt.%:

| | |
|---|---|
| alkyl polyglycol ether carboxylate, Tris salt | 2 - 4 |
| sodium lauryl sulphate | 1.5 - 3.5 |
| fatty acid amidopropylbetaine | 1.5 - 3 |
| protein hydrolysate | 0.3 - 1.5 |
| lauryl alcohol ethoxylate | 0.1 - 0.5 |
| polyquaternium (Merquat 550) | 0.2 - 2 |
| perfume | 0.5 - 2 |
| Na hexametaphosphate | 0 - 5 |
| NaCl | 2 - 10 |
| preserving agent | 0.05 - 0.5 |
| water | ad 100 |

**Revendications**

1. Gel de douche et shampoing présentant les particularités suivantes :
   a) le mélange est une solution aqueuse,
   b) elle contient une combinaison tensioactive neutralisée à base de carboxylate d'éther d'alkylpolyglycol, de sulfate d'alkyle et d'amidopropylbétaïne d'acide gras, dans un rapport d'environ 1,8-4,3/1,5-3,2/1,
   c) le degré d'éthoxylation moyen des carboxylates d'éther d'alkylpolyglycol est de 2-5,
   d) la teneur en agents tensioactifs est inférieure ou égale à 10 % en poids,
   e) le mélange contient des adjuvants et supports et électrolytes courants, à l'exception de substances solides insolubles et d'agents épaississants.

2. Gel de douche et shampoing suivant la revendication 1, dans lesquels le carboxylate d'éther d'alkylpolyglycol utilisé présente la formule générale I R-0-$(CH_2CH_2O)_n CH_2COOH$ (I), dans laquelle R représente un groupe alkyle ayant 10 à 16, en particulier 12 ou 14, atomes de C, et n représente en moyenne 2,5.

3. Gel de douche et shampoing suivant l'une des revendications 1 et 2, comprenant, comme agent de neutralisation, KOH, NaOH, de préférence du tris-(hydroxyméthyl)-aminométhane.

4. Gel de douche et shampoing suivant l'une ou plusieurs des revendications 1 à 3, caractérisé par une teneur en éthoxylate d'alcool laurylique.

5. Gel de douche et shampoing suivant une ou plusieurs des revendications 1 à 4, caractérisé en ce qu'il comprend des agents de conditionnement, tels que des hydrolysats de protide et/ou des polymères d'acrylamide.

6. Gel de douche et shampoing suivant une ou plusieurs des revendications 1 à 5, caractérisé en ce qu'il comprend un électrolyte, en particulier du chlorure de sodium, ou un polyphosphate, en particulier de l'hexamétaphosphate de Na, comme agent épaississant.

7. Gel de douche et shampoing suivant une ou plusieurs des revendications 1 à 6, caractérisé par la composition suivante, en % en poids :

| | | | |
|---|---|---|---|
| Sel tris de carboxylate d'éther d'alkylpolyglycol | 2 | – | 4 |
| Laurylsulfate de sodium | 1,5 | – | 3,5 |
| Amidopropyl-bétaïne d'acide gras | 1,5 | – | 3 |
| Hydrolysat de protide | 0,3 | – | 1,5 |
| Ethoxylate d'alcool laurylique | 0,1 | – | 0,5 |
| Polyquaternium (Merquat 550) | 0,2 | – | 2 |
| Parfum | 0,5 | – | 2 |
| Hexamétaphosphate de Na | 0 | – | 5 |
| NaCl | 2 | – | 10 |
| Agent de conservation | 0,05 | – | 0,5 |
| Eau | ad 100 | | |